(19) Europäisches Patentamt / European Patent Office / Office européen des brevets

(11) **EP 4 216 810 B1**

(12) **FASCICULE DE BREVET EUROPEEN**

(45) Date de publication et mention
de la délivrance du brevet:
**06.11.2024 Bulletin 2024/45**

(21) Numéro de dépôt: **21794188.9**

(22) Date de dépôt: **23.09.2021**

(51) Classification Internationale des Brevets (IPC):
**A61B 5/0533** (2021.01)    **A61B 5/16** (2006.01)
**A61B 5/00** (2006.01)    **A61B 5/024** (2006.01)

(52) Classification Coopérative des Brevets (CPC):
**A61B 5/0533; A61B 5/165; A61B 5/4035;**
A61B 5/02416; A61B 5/721

(86) Numéro de dépôt international:
**PCT/IB2021/058697**

(87) Numéro de publication internationale:
**WO 2022/064425 (31.03.2022 Gazette 2022/13)**

(54) **ÉQUIPEMENT ÉLECTRODERMAL**

**ELEKTRODERMALES GERÄT**

**ELECTRODERMAL APPARATUS**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **23.09.2020 FR 2009668**

(43) Date de publication de la demande:
**02.08.2023 Bulletin 2023/31**

(73) Titulaire: **OVOMIND SA**
**1228 Plan-les-Ouate (CH)**

(72) Inventeur: **FRACHI, Yann**
**13012 Marseille (FR)**

(74) Mandataire: **IP Trust**
**2, rue de Clichy**
**75009 Paris (FR)**

(56) Documents cités:
EP-B1- 4 031 005     WO-A1-2017/202626
FR-A1- 3 063 425     KR-A- 20160 085 577
US-A1- 2013 183 646

- RUI GUO ET AL: "Pervasive and unobtrusive emotion sensing for human mental health", PERVASIVE COMPUTING TECHNOLOGIES FOR HEALTHCARE (PERVASIVEHEALTH), 2013 7TH INTERNATIONAL CONFERENCE ON, IEEE, 5 May 2013 (2013-05-05), pages 436 - 439, XP032439656, ISBN: 978-1-4799-0296-5, DOI: 10.4108/ICST.PERVASIVEHEALTH.2013.252133
- JORN BAKKER ET AL: "What's Your Current Stress Level? Detection of Stress Patterns from GSR Sensor Data", DATA MINING WORKSHOPS (ICDMW), 2011 IEEE 11TH INTERNATIONAL CONFERENCE ON, IEEE, 11 December 2011 (2011-12-11), pages 573 - 580, XP032100119, ISBN: 978-1-4673-0005-6, DOI: 10.1109/ICDMW.2011.178

**Description**

**Domaine de l'invention**

La présente invention concerne le domaine de la mesure de la réponse galvanique de la peau et de l'activité électro-cutanée, et en particulier des dispositifs portables et des procédés pour mesurer l'activité électrodermique de la peau d'un utilisateur.

L'activité électrodermale est une activité électrique biologique enregistrée à la surface de la peau et reflétant l'activité des glandes de la sudation et du système nerveux autonome et par conséquent, entre autres, de la perception de l'individu et de son comportement involontaire plutôt que celui d'une réponse qu'il souhaite donner.

L'étude scientifique du réflexe cutané galvanique a commencé au début des années 1900. Le psychologue C.G. Jung est parmi les premiers à utiliser son enregistrement en psychanalyse et il relate cette expérience dans son ouvrage « Studies in Word Analysis » (Études sur l'analyse du mot) publié en 19062. Wilhelm Reich a également étudié la réponse électrodermique dans des expériences menées à l'Institut de psychologie de l'Université d'Oslo, en 1935 et 1936, dans le but de confirmer l'existence d'une charge bio-électrique derrière son concept de « courants végétatifs de plaisir ».

Dans les années 1960 et jusqu'à la fin des années 1970, la réponse psychogalvanique a été utilisée dans tous les domaines de la psychologie pour des recherches très variées. Les phénomènes psycho-galvaniques sont liés à l'activité du système nerveux sympathique, et se manifestent par la variation de la résistance électrique de la peau, à la suite d'une émotion.

Depuis, de nombreux travaux ont porté sur l'exploitation des signaux GSR (Galvanic Skin Response) ou RED (résistance électrodermale) représentatif de la variation de la résistance électrique de la peau provoquée par l'activité des glandes sudoripares suite à une excitation sensorielle, appelée réflexe psychogalvanique, pour des applications diverses telles que les détecteurs de mensonge, l'analyse des émotions ou le pilotage de jeux vidéo par des interfaces homme-machine nouvelles.

**Etat de la technique**

On connaît dans l'état de la technique le brevet FR3063425 proposant un système de détermination d'une émotion d'un utilisateur comprenant un capteur d'activité électrodermale de l'utilisateur, un premier circuit adapté pour: a/ détecter des moments émotionnels forts de l'utilisateur en fonction d'une analyse d'informations issues du capteur (d'activité électrodermale, b/ en cas de détection d'un moment émotionnel fort à l'étape /a/, demander à l'utilisateur de classifier ledit moment émotionnel fort.

Cette solution nécessite de un apprentissage à l'initiative de l'utilisateur, qui doit identifier un « moment émotionnel fort » et ensuite le classifier. Il est bien évident que cette solution aboutit à des comportements erratiques voire fantaisiste, et en aucune façon reproductible et fiable car elle dépend de la subjectivité de l'utilisateur, de sa capacité à mener à bien cet apprentissage, et la nature des évènements auquel est exposé l'utilisateur pendant cette phase d'apprentissage.

La demande de brevet WO2017202626 concerne un capteur destiné à mesurer la conductance de la peau. Un amplificateur est utilisé pour convertir la conductance de la peau en une tension de sortie analogique qui est ensuite convertie dans le domaine numérique, de sorte que l'augmentation de la conductance de la peau tonique et la réponse de conductance de la peau phasique sont obtenues dans le domaine numérique. L'amplificateur a un gain logarithmique non linéaire, avec un gain décroissant pour augmenter des valeurs de conductance de la peau. Le capteur permet de détecter à la fois l'augmentation des signaux toniques et phasiques sur une large plage de conductance de la peau. Il permet une utilisation optimale du convertisseur analogique-numérique de sorte qu'une résolution inférieure et donc un convertisseur moins cher peuvent être utilisés.

La demande de brevet US 2013/183646 décrit un appareil permettant d'utiliser de manière interopérable plusieurs données de biocapteur fixé sur le doigt de l'utilisateur. Ces capteurs peuvent être des capteurs à réponse électrodermique (EDR), ou des signaux de photopléthysmographe (PPG), ou de température, ou d'accélération sur trois axes, et des combinaisons de ceux-ci. La carte biométrique enregistre et traite les signaux des capteurs et les communique à un dispositif mobile qui utilise de manière interopérable plusieurs informations de capteur pour déterminer les aspects de l'état émotionnel de l'utilisateur.

On connaît aussi l'article « RUI GUO ET AL: "Pervasive and unobtrusive émotion sensing for human mental health", PERVASIVE COMPUTING TECHNOLOGIES FOR HEALTHCARE (PERVASIVEHEALTH), 2013 7TH INTERNATIONAL CONFERENCE ON, IEEE, 5 mai 2013 (2013-05-05), pages 436-439, XP032439656, DOi: 10.4108/ ICST.PERVASIVEHEAL TH.2013.252133, ISBN: 978-1 -4 799-0296-5.

Un autre article JORN BAKKER ET AL: "What's Your Current Stress Level? Détection of Stress Patterns from GSR Sensor Data", DATA MINING WORKSHOPS (ICDMW), 2011 IEEE 11 TH INTERNATIONAL CONFERENCE ON, IEEE, 11 décembre 2011 (2011-12-11), pages 573-580, XP032100119, DOi: 10.1109/ ICDMW.2011.178, ISBN: 978-1-4673-0005-6 concerne les phénomènes de détection de stress.

On connaît aussi le brevet KR20160085577 décrivant une autre solution pour déterminer un état psychologique selon un mode de réalisation de la présente invention comprend : une unité d'acquisition de bio-signal pour acquérir un premier bio-signal et un deuxième bio-signal d'un utilisateur généré par un premier stimulus ; et une unité de détermination d'état psychologique pour extraire une première caractéristique et une deuxième caractéristique en analysant le premier bio-signal et le deuxième bio-signal et en déterminant l'état psychologique de l'utilisateur sur la base de la première caractéristique et de la deuxième caractéristique. Le premier bio-signal et le deuxième bio-signal sont des bio-signaux indiquant des changements dans les nerfs autonomes mesurés par différents bio-capteurs. Par exemple, le premier bio-signal est un signal PPG mesuré par un capteur d'onde de pouls, et le second bio-signal est un signal GSR mesuré par un capteur de conductivité cutanée.

Solution apportée par l'invention

Afin de remédier aux inconvénients des solutions de l'art antérieur, la présente invention concerne selon son acception la plus générale un système conforme à la revendication 1.

Avantageusement, lesdites électrodes sont constituées par des plots formés par une feuille d'acier estampée et étamée.

Optionnellement, l'une au moins desdites électrodes présente un logement apte à recevoir un capteur additionnel.

Selon une variante, ledit équipement electrodermal comporte en outre un capteur de température.

Avantageusement, le capteur de température est positionné sur une autre électrode ou sonde en acier dédiée. L'épaisseur de cette sonde dédiée est spécifique afin d'éviter trop d'inertie dans le signal et de perdre des micro-variations importantes dans le suivi des réactions émotionnelles.

L'équipement electrodermal comporte en outre un capteur de rythme cardiaque, de préférence un capteur de rythme cardiaque délivrant des signaux physiologiques par photopléthysmographie (PPG). Le capteur cardiaque est positionné sous une vitre (PPG) et n'est pas lié à l'électrode métallique.

Les techniques applicables au signal PPG comprennent :

- les techniques comme la SVD et l'ICA qui n'exploitent qu'un seul canal PPG et fournissent donc des informations relativement « pauvres » mais peuvent être tester très rapidement.
- L'Analyse en Séries de Fourier avec une détermination automatique du nombre de coefficients à retenir (comme pour l'ICA) est une technique envisageable mais assez complexe en raison du bruit induit par les mouvements pouvant dans certaines circonstances transitoires être plus puissant que le signal PPG.
- Le traitement de type Periodic Moving Average Filter peut être efficace mais va induire beaucoup de latence dans le signal.
- Le traitement de type Wavelet Denoising constitue une alternative efficace aux autres méthodes.
- La technique « Active Noise Cancellation » basée sur un filtrage adaptatif est la méthode qui me semble la plus approprié à notre approche multimodale et peut être couplée à l'Empirical Mode Décomposition pour améliorer encore le débruitage.

L'invention concerne aussi un système comprenant un capteur electrodermal selon l'une au moins des variantes précédentes et un calculateur délivrant au moins une donnée numérique représentatives d'un état émotionnel, caractérisé en ce que ledit calculateur exécute un programme commandant :

- Le traitement des données numériques de conductance fournies par ledit processeur sur une fenêtre temporelle glissante dont le résultat fourni une valeur $S_{arousal}$.

Il comporte en outre une étape de traitement de ladite deuxième série de signaux de rythme cardiaque consiste en un filtrage de passe-bande des fréquences comprises entre 0,04 et 0,26 Hz et de détection de pics et de mesure temporelle inter-pic RR, sur une fenêtre temporelle glissante dont le résultat fourni une valeur $S_{valence}$.

Description détaillée d'un exemple non limitatif de l'invention

[Fig.1] la [Fig.1] représente une vue en perspective d'une électrode selon l'invention
[Fig.2] la [Fig.2] représente une vue partielle d'un bracelet muni de deux capteurs selon l'invention.

Architecture matérielle

L'invention concerne un dispositif permettant l'acquisition de signaux physiologiques par l'intermédiaire de deux électrodes (ou plus) en contact avec l'épiderme d'un utilisateur, et l'exploitation de différents types de signaux pour l'estimation d'un état émotionnel fiable et indépendant d'artefacts tels que les mouvements de l'utilisateurs. L'espacement entre les électrodes est important et déterminé, afin que la quantité de peau traversée par le signal soit suffisante pour avoir un signal exploitable. Typiquement un espacement de 2 à 20 millimètres est adapté.

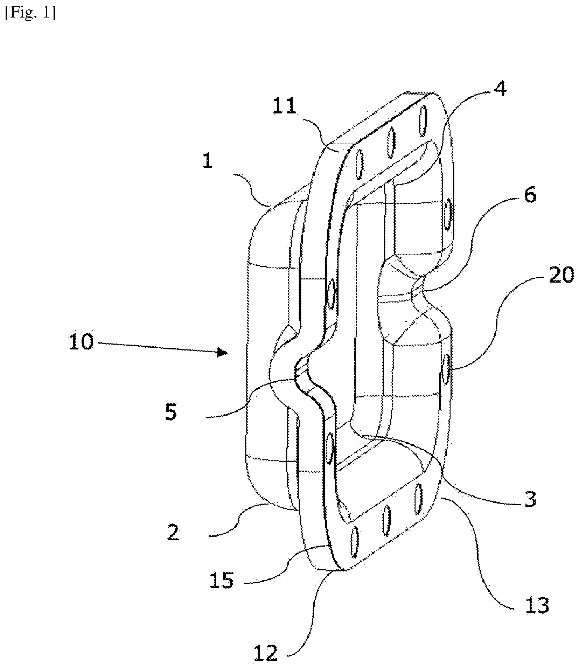

L'invention met en oeuvre deux électrodes métalliques dont la [Fig.1] est un exemple de vue en perspective.

Une électrode est formée par estampage d'une feuille en acier inoxydable, par exemple INOX 316L d'une épaisseur de 0,5 millimètres pour former un plot électrique (10) d'une hauteur de 2 millimètres, et de 11 millimètres de long, de 8 millimètres de large, de section rectangulaire à coins (1 à 4) arrondis avec un rayon de courbure de 1,5 millimètres, prolongé par une bordure (15) de section rectangulaire d'une largeur de 1,5 millimètres, avec des coins (11 à 13) arrondis avec un rayon de courbure de 3 millimètres. Le rayon de courbure des arêtes est de 0,3 millimètres. La bordure est percée de trous (20) d'une section de 0,8 millimètres pour la fixation sur un bracelet ou une platine support. Des échancrures (5, 6) de section semi-circulaire d'un rayon de 1,1 millimètres permettent le passage de câbles électriques. La surface extérieure de l'électrode est en contact avec l'épiderme et permet, avec une seconde électrode placée à proximité, la mesure de la conductivité électrique observée à la surface de la peau de l'utilisateur et fournissant un signal représentatif de l'activité électrodermale (EDA)

L'électrode (10) peut optionnellement intégrer une pluralité de capteurs destinés à l'obtention de mesures de signaux physiologiques associés aux émotions de l'utilisateur, par exemple :

- un capteur apte à la mesure d'un rythme cardiaque de l'utilisateur via une lumière formée dans l'électrode métallique, et une vitre fermant ladite lumière ;
- un capteur apte à la mesure de la température superficielle de la peau fixé sur le fond par une colle thermique ;
- un capteur constitué par un accéléromètre trois-axes ou multi-axes tels qu'un module inertiel 9 axes apte à permettre la mesure de mouvements sur un membre de l'utilisateur, par exemple un accéléromètre piézoélectrique ou un accéléromètre trois axes MEMS et un gyroscope (rotation de poignet).

Les signaux fournis par l'accéléromètre sont représentatifs des mouvements de l'utilisateur et peuvent être exploiter pour filtrer les signaux physiologiques afin d'éliminer les signaux transitoires lors des mouvements brusques, pouvant altérer la conduction électrique ou le positionnement de l'électrode sur l'épiderme. Cette fonctionnalité de filtrage des signaux bruités est particulièrement importante lorsque les signaux physiologiques sont analysés sur une fenêtre temporelle longue, de plusieurs secondes car la prise en compte de signaux anormaux fausserait gravement les évaluations produites par ces analyses temporelles.

Détail du bracelet

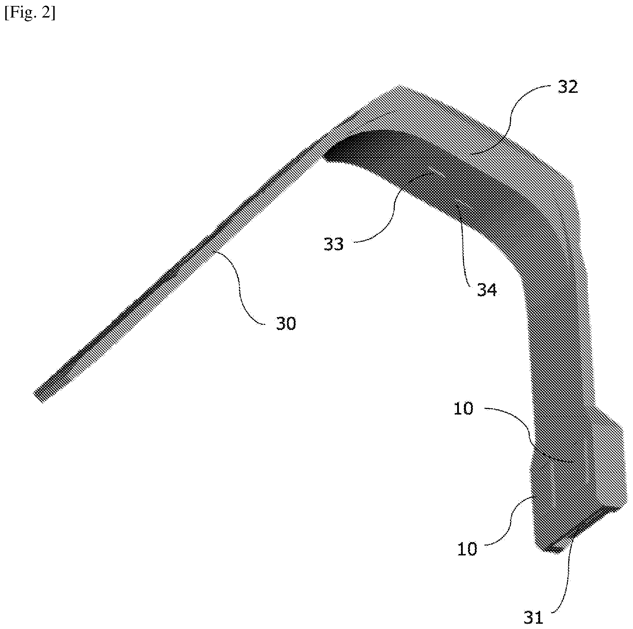

La [Fig.2] représente un exemple de montage de deux électrodes (10) de 8x5 millimètres et une épaisseur de 0,5 millimètres, disposées transversalement, avec un écartement de 10 millimètres, sur un bracelet (30). Le bracelet présente un fermoir (31) et un logement (32) pour recevoir les circuits électroniques. L'ensemble peut être surmoulé pour assurer une insensibilité à l'humidité et aux poussières.

Le fond de cette zone présente un capteur de température (33) et un capteur optique (34) comprenant une photodiode et des leds pour capter des signaux liés à la circulation sanguine, par exemple le rythme cardiaque et le taux d'oxygénation.

Les électrodes sont espacées de et polarisées par un circuit électronique mettant en oeuvre un circuit convertisseur ADC LTC2487 ANALOG DEVICES (nom commercial) comportant deux entrées différentielles et assurant l'échantillonnage sur 16 bits ainsi qu'un circuit de compensation en température. Il est aussi possible d'utiliser l'ADC du microcontrôleur Nordic nRF52 ce qui permet d'avoir deux modalités de signal avec les mêmes composants. Ce capteur permet de mesurer la résistance de la peau via deux ponts diviseurs en entrée d'un convertisseur analogique-numérique (ADC) en différentiel depuis un point commun et une mesure de la tension induite par la variation de la résistance du corps humain, typiquement entre 500 et 300 Ohm.

Capteurs équipant le bracelet

Le bracelet (30) peut être équipé de plusieurs capteurs :

a. Un capteur galvanique utilisant les deux électrodes (10) fournissant un signal « glavanic skin response » échantillonné à 8 Hz, exploités par le calculateur pour caractériser le score Arousal et le niveau de vigilance

b. Un accéléromètre 3 axes échantillonné à 64 Hz, exploités par le calculateur pour caractériser les pas et les chutes
c. Un capteur sanguin (34) échantillonné à 50 Hz, exploités par le calculateur pour caractériser le score de valence, la reconnaissance biométrique, le rythme cardiage et le niveau de stress
d. Un capteur de température (33) échantillonné à 1 Hz pour la mesure de la température du corps.

Le bracelet comporte un calculateur pour le prétraitement des données qui sont ensuite transmise sous forme de paquets numériques via un protocole radiofréquence par un module radio et une antenne.

Traitement des signaux échantillonnés

Différents types de traitement peuvent être envisagés pour fournir les analyses émotionnelles.

L'Analyse en Séries de Fourier, est une solution usuelle impliquant toutefois une contrainte relative à la détermination automatique du nombre de coefficients à retenir. Cette contrainte est assez complexe comme le bruit induit par les mouvements risque d'être plus puissant que le signal PPG sans pour autant être présent en continu. Toutefois, l'utilisation d'une solution de débruitage basée sur le filtrage des signaux en fonction des informations fournies par l'accéléromètre permet de réduire cette difficulté.

Le capteur fournit un signal représentatif d'un paramètre passif ou endosomatique correspondant au niveau de conductance dermique (skin conductance level SCL) ou d'un paramètre actif ou exosomatique correspondant au niveau de la réponse de conductance dermique (skin conductance responses SCR). Ces paramètres permettent de déterminer l'activité électrodermale (AED) trouvant son origine dans des caractéristiques de la membrane épidermique, et dans l'activité sudoripare de type eccrine sous contrôle des systèmes nerveux autonome et central.

On distingue deux méthodes d'enregistrement.

La première méthode dite endosomatique traduit les différences de potentiels générées par les membranes cutanés et conduit à la mesure du potentiel électrodermal. Dans ce cas, le capteur est un capteur de conductivité de la peau associé à un convertisseur courant-tension, par exemple un capteur de résistivité de la peau, muni d'une paire d'électrodes en acier noble.

La seconde méthode dite exosomatique traduit les variations d'un courant appliqué à la peau dont les caractéristiques peuvent conduire à la mesure de divers signaux électrodermaux dont la mesure de conductance cutanée, la plus couramment utilisée dans la littérature. Chacun des signaux électrodermaux se subdivise en une composante tonique et une composante phasique.

La première identifie les variations lentes du signal électrodermal alors que la seconde correspond aux variations rapides du signal communément appelées réponses électrodermales. De ces mesures phasiques peuvent être extraits différents paramètres de mesure tels que la fréquence, la latence ou l'amplitude des réponses électrodermales. Les origines ainsi que la variabilité des paramètres de mesure de l'activité électrodermale font de cette activité une mesure sensible aux changements de notre environnement et à différents processus mentaux sous contrôle du système nerveux central tels que l'émotion, la motivation ou encore l'attention, et la charge mentale.

Chaque capteur est associé à un circuit de prétraitement réalisant optionnellement un traitement analogique (préemplification, filtration, délivrance d'un signal d'excitation), de numérisation (échantillonnage, filtrage numérique optionnel, stockage dans une mémoire tampon,...) pour délivrer à un calculateur des signaux numériques exploités pour déterminer le couple de valeurs représentatives de l'état émotionnel.

Traitements des signaux physiologiques

Les signaux fournis par un couple d'électrode sont échantillonnés à une fréquence de 64 Hz, voire 130 Hz, et filtrés en amplitude et en fréquence pour supprimer les signaux aberrants. Ces signaux constituent des informations environnementales venant compléter les signaux associés aux émotions, par exemple pour donner un contexte de mode de déplacement et/ou de chute délivrés par l'accéléromètre.

Les signaux fournis par le capteur (10) de conductivité électrique sont échantillonnés à une fréquence de 8 Hz et ensuite traité pour le calcul du score d'arousal et le niveau de vigilance.

Les signaux fournis par le capteur de rythme cardiaque de l'utilisateur sont échantillonnés à une fréquence de 100 Hz et exploités par le calculateur pour la détermination du score de valence, ainsi que pour la reconnaissance biométrique et l'estimation du niveau de stress.

Le capteur de mesure de la température de la peau est échantillonné à une fréquence basse de l'ordre de 1Hz et complète les informations permettant de caractériser l'état émotionnel.

Exemple de mise en oeuvre particulier

Un exemple de mise en oeuvre consiste à équiper le patient d'un bracelet connecté sans fil équipé de trois capteurs physiologiques (un seul capteur peut suffire) mesurant la conductance électrodermale (notée GSR pour galvanic skin response) à une cadence 8Hz, l'activité cardiaque (notée PPG pour photoplethysmography) à une cadence 50Hz, la température corporelle (notée SKT pour skin température) à une cadence 1Hz et d'un ou plusieurs capteurs accélérométriques et gyroscopiques (notés ACC) à une cadence de 50Hz sont utilisés pour enregistrer de façon synchrone les données et les time stamps correspondants.

Les données GSR et PPG sont transmises à un terminal mobile qui effectue les calculs pour l'identification en temps réel de l'état émotionnel. Les données GSR et PPG sont stockées dans des tampons de calcul dont les durées varient selon les variables calculées.

Dans chaque mémoire tampon, le traitement du signal est effectué avant l'extraction des différentes variables utilisées pour l'analyse d'identification de l'état émotionnel :

Signal GSR : Un filtrage passe-bande (butterworth de 4$^{\text{ème}}$ ordre) est appliqué au signal avec une bande passante de 0.05 - 1Hz.
Signal PPG : Un filtrage passe-bande (butterworth de 4$^{\text{ème}}$ ordre) est appliqué au signal avec une bande passante de 0.5 - 5Hz.

Les variables utilisées pour l'analyse d'identification de l'état émotionnel sont ensuite extraites des signaux traités. La variable *Arousal* est obtenue dans une mémoire tampon de calcul de 20 secondes à partir de la puissance spectrale normalisée du signal GSR calculée sur la bande 0.045 - 0.25Hz par une transformation Hilbert-Huang.

Exemple de traitement

La variable *Mdiff* est enregistrée dans un tampon de calcul de 2 secondes à partir de la moyenne de la valeur absolue de la dérivée première du signal GSR.

La variable *Valence* est obtenue dans un tampon de calcul de 60 secondes en calculant le ratio de cohérence cardiaque. Pour cela, une détection des pics dans le signal PPG est réalisée à partir d'une fonction dédiée pour en déduire les intervalles de temps pic à pic notés intervalles RR. Puis, le rythme cardiaque noté *BPM* est calculé à partir des intervalles RR.

A partir du signal *BPM,* le pic maximum du spectre de puissance est identifié sur la bande 0.04 - 0.26Hz (la gamme de fréquence au sein de laquelle la cohérence peut être observée). La puissance de ce pic notée *Peak Power* est ensuite déterminé en calculant l'intégrale sur une fenêtre de 0.030Hz de large, centrée autour du pic. La puissance totale sur la bande 0.0033 - 0.4Hz du signal BPM notée *Total Power* est ensuite calculée. Le niveau de valence normalisé est obtenu par le calcul suivant :

[Math.1]

$$Valence = \frac{Peak\ Power}{Total\ Power - Peak\ Power} \quad (1)$$

Toutes les secondes, les nouvelles valeurs GSR et PPG enregistrées par le bracelet permettent de calculer les nouvelles valeurs *Arousal, Mdiff* et *Valence.*

*Mdiff* est stockée en mémoire la dernière minute pour permettre une calibration dynamique du système de détection des variations ponctuelles du niveau d'excitation physiologique. Un coefficient de pondération est appliqué à ces données de calibration pour rendre la contribution des valeurs les plus récentes plus importante lors de la calibration. Il est ensuite possible de calculer les seuils dynamiques permettant de classifier respectivement la variable *Mdiff.* Le calcul des seuils peut être détaillé de la façon suivante :

[Math.2]

$$Seuil_{(t)} = \frac{Max\left(Mdiff\begin{bmatrix} x_1 \\ \vdots \\ x_n \end{bmatrix}\right) + Moyenne\left(Mdiff\begin{bmatrix} x_1 \\ \vdots \\ x_n \end{bmatrix}\right)}{2} \quad (1)$$

$$Mdiff \begin{bmatrix} x_1 \\ \vdots \\ x_n \end{bmatrix}$$

Avec *Seuil*$_{(t)}$ la valeur du seuil dynamique à l'instant *t* et (à droite) les valeurs de la variable *Mdif f* sur toute la durée de la période de calibration.

Chaque seconde, une nouvelle valeur de *Seuil* est obtenue et comparée à *Mdif f*. Si *Mdif f* est supérieure à sa valeur seuil, alors une réaction émotionnelle est détectée.

Autres modes de traitement

Le traitement selon la solution « Periodic Moving Average Filter » peut être efficace mais induit beaucoup de latence dans le signal.

Le traitement de type « Wavelet Denoising » est une solution pertinente, ainsi que le traitement de type « Active Noise Cancellation » basée sur un filtrage adaptatif. C'est la méthode la plus approprié à une approche multimodale et peut être couplée à un traitement de type « Empirical Mode Décomposition » pour améliorer encore le débruitage.

Exemples spécifiques de traitement

Le but de l'invention est de fournir automatiquement et sans intervention d'un humain des signaux numériques $S_{arousal}$ et/ou $S_{valence}$ représentatifs de l'état émotionnel d'une personne.

La reconnaissance efficiente des émotions à partir de l'activité physiologique humaine peut passer par l'utilisation d'un modèle émotionnel simple. En effet, les émotions peuvent être projetées dans un espace multidimensionnel, le plus commun étant le plan valence-éveil. Le niveau de valence représente la positivité et la négativité d'une émotion alors que le niveau d'éveil (arousal en anglais) décrit l'intensité de l'émotion. Ces deux composantes émotionnelles sont exprimées au niveau physiologique.

Lors d'un stress, le système nerveux sympathique prédomine et conduit à une élévation du niveau d'éveil physiologique. Une accélération de la fréquence cardiaque ou une accélération de l'intervalle inter-battement (IBI Interbeat interval) est caractéristique de cet état. Le débruitage est d'autant plus important pour cet indicateur dont découle la Valence. La lumière est plus sensible au mouvements (PPG).

Au repos, au contraire, le système nerveux parasympathique s'active traduisant une diminution de l'état d'éveil physiologique et de la fréquence cardiaque. Par ailleurs, l'alternance des accélérations et des décélérations de la fréquence cardiaque devient régulière et cohérente (état de cohérence cardiaque) dans les états de bien-être, de calme, de maîtrise (valence émotionnelle positive) alors que dans les états de stress, d'anxiété, de colère (valence émotionnelle négative), le tachogramme correspondant au couple $S_{arousal}$ et $S_{valence}$ devient irrégulier, son tracé chaotique et sa magnitude va diminuer.

En extrayant du signal PPG le niveau de cohérence du rythme cardiaque, il devient possible d'obtenir un indicateur robuste du niveau de valence émotionnelle et de calculer des seuils dynamiques au-delà desquels ce niveau valence change de façon significative.

Une fois le niveau de valence estimé, il est alors possible de vérifier le niveau d'éveil en contrôlant dans le domaine spectral le niveau d'activation physiologique à partir du signal GSR pour en déduire en temps réel l'état émotionnel de l'individu et le communiquer au système multimédia avec lequel ce dernier interagit.

On peut caractériser l'état émotionnel selon le tableau suivant :

[Tableaux1]

| | $S_{valence}$ | | + | | | |
|---|---|---|---|---|---|---|
| + | | | CRISPE | ALERTE | | |
| | | NERVEUX | | | EXITE | |
| | CONTRARIE | | | | | |
| $S_{arousal}$ | TRISTE | | | | | HEUREUX |
| | DREPRESSIF | | | | CONTENT | |
| | | ENNUYE | | SEREIN | | |
| - | | | RELAXE | | | |

Lors d'un stress, le système nerveux sympathique prédomine et conduit à une élévation du niveau d'éveil physiologique. Une accélération de la fréquence cardiaque est caractéristique de cet état. Au repos, au contraire, le système nerveux parasympathique s'active traduisant une diminution de l'état d'éveil physiologique et de la fréquence cardiaque. Par ailleurs, l'alternance des accélérations et des décélérations de la fréquence cardiaque devient régulière et cohérente (état de cohérence cardiaque) dans les états de bien-être, de calme, de maîtrise (valence émotionnelle positive) alors que dans les états de stress, d'anxiété, de colère (valence émotionnelle négative), le tachogramme devient irrégulier, son tracé chaotique et sa magnitude va diminuer.

En extrayant du signal PPG le niveau de cohérence du rythme cardiaque, il devient possible d'obtenir un indicateur robuste du niveau de valence émotionnelle et de calculer des seuils dynamiques au-delà desquels ce niveau valence change de façon significative.

Une fois le niveau de valence estimé, il est alors possible de vérifier le niveau d'éveil en contrôlant dans le domaine spectral le niveau d'activation physiologique à partir du signal GSR pour en déduire en temps réel l'état émotionnel de l'individu et le communiquer au système multimédia avec lequel ce dernier interagit.

Apprentissage des critères de caractérisation

Pour construire un modèle de caractérisation, l'invention propose une variante mettant en oeuvre une étape préparatoire d'apprentissage supervisé.

Cette solution consiste à proposer à un panel d'usagers équipé d'un dispositif d'acquisition des données physiologiques susvisé, des plans d'expériences formés par une succession de séquences vidéos labélisée émotionnellement associées chacune à un descripteur numérique ID (t), et d'enregistrer les couples de signaux $S_{arousal}$ et $S_{valence}$ et leur évolution dans le temps, pour chacun des membres du panel.

Ces données structurées ($S_{arousal}$ et $S_{valence}$ (t) ; ID (t)) pour chacun des membres du panel sont ensuite injectés dans un réseau de neurones, pour élaborer un modèle de caractérisation.

Les participants seront équipés d'un bracelet connecté conforme à l'invention équipés de trois capteurs physiologiques mesurant l'activité cardiaque (notée PPG pour photoplethysmography), la température corporelle (notée SKT pour skin température) et conductance électrodermale (notée GSR). Les bracelets communiquent avec un ordinateur portable d'acquisition permettant d'enregistrer de façon synchrone les données et l'horodatage correspondant avec une fréquence d'acquisition de 50Hz, 1Hz et 4Hz pour le PPG, la SKT et le GSR, respectivement pour le bracelet connecté ou montre connectée et avec une fréquence d'acquisition de 64Hz, 4Hz et 4Hz pour le PPG, la SKT et le GSR.

Un système de réalité virtuelle HTC Vive sera utilisé pour afficher les stimuli sélectionnés pour induire une réaction émotionnelle et permet d'avoir une immersion supplémentaire (inédit en protocole de stimulation émotionnel)

Design expérimental

Pour chaque participant, les données seront enregistrées sur une seule session de vingt minutes. Le plan d'expérience est : Sn (participants) * V6 (six vidéos émotionnelles).

Chaque vidéo correspond à un extrême émotionnel :

- Vidéo 1: Repos (40s) - Phase d'induction émotionnelle de type tristesse (30s) - Post-effet (30s)
- Vidéo 2: Repos (40s) - Phase d'induction émotionnelle de type joie (30s) - Post-effet (30s)
- Vidéo 3: Repos (40s) - Phase d'induction émotionnelle de type dégoût (30s) - Post-effet (30s)
- Vidéo 4: Repos (40s) - Phase d'induction émotionnelle de type peur (30s) - Post-effet (30s)
- Vidéo 5: Repos (40s) - Phase d'induction émotionnelle de type neutre (30s) - Post-effet (30s)
- Vidéo 6: Repos (40s) - Phase d'induction émotionnelle de type relaxation (30s) - Post-effet (30s)

La phase repos constituera une période de référence pour initialiser le calcul des variables physiologiques. Pour chaque participant, l'ordre de présentation des vidéos sera aléatoire afin d'éviter tout effet d'ordre. De plus, afin d'enrichir le jeu de données, deux vidéos seront disponibles pour les émotions de type peur et joie. Pour chaque participant, le choix de la vidéo utilisée pour chacune de ces deux émotions sera aléatoire.

Procédure d'acquisition des données

Chaque participant est d'abord équipé d'un ou plusieurs bracelets connectés et d'un système de réalité virtuelle lui permettant de s'isoler des stimulations extérieures et d'optimiser son focus attentionnel. L'expérimentateur vérifie ensuite la qualité des signaux physiologiques. Chaque participant aura pour instruction générale de visualiser six vidéos d'une durée de 30 secondes. Lors des 40 secondes précédent la vidéo et des 30 secondes suivant la vidéo, la consigne donnée sera de rester calme et immobile. Lorsque toutes les vidéos ont été visualisées, l'expérimentateur aide le participant à enlever le casque de réalité virtuelle et le bracelet puis réalise un débriefing pour vérifier que tout s'est bien passé.

Analyse des données

Pour chaque participant, les données physiologiques enregistrées seront prétraitées de la façon suivante :

Pour le signal PPG, les sauts de signal seront corrigés à l'aide d'une fonction dédiée. Un filtrage passe-bande (butterworth de 4ème ordre) sera ensuite appliqué au signal avec une bande passante de 0.5 - 5Hz puis le signal sera normalisé à l'aide d'une transformée d'Hilbert et lissé à l'aide d'une fenêtre gaussienne de 16 secondes. En ce qui concerne le signal SKT, un filtrage passe-bas (butterworth de 4ème ordre) sera appliqué au signal avec une fréquence de coupure à 0.05Hz. Enfin, un filtrage passe-bande (butterworth de 4ème ordre) sera appliqué au signal GSR avec une bande passante de 0.05 - 3Hz. Toutes ces variables constitueront les données d'entrée des algorithmes de classification émotionnelle.

Représentation graphique des résultats des traitements

Toutes les secondes, les variables obtenues sont représentées par le système d'affichage de l'état émotionnel détecté (noté overlay) de la façon suivante :

Le diamètre du cercle correspond à la valeur normalisée de la valeur $S_{Arousal}$. Plus le diamètre est important, plus le niveau d'éveil est élevé.

La couleur du cercle correspond à la valeur normalisée de la valeur $S_{Valence}$. Lorsque la couleur tend vers le vert, le niveau de valence est plus élevé. Lorsque la couleur tend vers le rouge, le niveau de valence est plus faible.

Lorsqu'une une réaction émotionnelle est détectée à partir du paramètre d'évolution $Mdiff$, le contour du cercle s'anime. La valeur du rythme cardiaque s'actualise au centre du cercle.

L'état émotionnel est communiqué au système multimédia avec lequel l'individu interagit ([Fig.2]). Il est important de noter que le dock/mobile prévoit la mise à jour du bracelet d'une part et des méthodes de calculs des valeurs et des niveaux de détection d'autre part via une connexion internet.

Applications

Le procédé mis en oeuvre par un système selon l'invention permet de délivrer des signaux de commande pour le pilotage d'un équipement tel qu'un robot, notamment un robot empathique ou de commande de paramètres fonctionnels d'un équipement électronique tel que le niveau sonore, lumineux, le rythme...

Ces signaux permettent également de commander l'adaptation de la vitesse d'un véhicule de transport individuel / collectif et la gestion des agents de sécurité, de contrôle, pilotes, conducteurs mais aussi la réduction de nombre d'informations en situation délicate en pilotage (avion de chasse ou F1).

**Revendications**

1. Système (30) comprenant un calculateur, un capteur de rythme cardiaque (34) et un capteur électrodermal comprenant au moins deux électrodes (10) configurées pour être chacune en contact avec une zone dermale, et un circuit électronique comportant un convertisseur analogique-numérique et un processeur configuré pour exécuter des instructions exécutables par ordinateur pour polariser lesdites électrodes (10) pour mesurer le courant circulant entre des paires d'électrodes, appliquer un prétraitement numérique aux signaux électriques mesurés et déterminer la conductance de la couche dermale, ledit capteur comprenant en outre un accéléromètre, délivrant des signaux électriques audit processeur, ledit prétraitement prenant en compte le niveau desdits signaux délivrés par ledit accéléromètre **caractérisé en ce que** ledit calculateur exécute un programme commandant :

   - le traitement des données numériques de conductance fournies par ledit processeur sur une première fenêtre temporelle glissante dont le résultat fourni une valeur $S_{arousal}$ représentative de l'intensité d'une émotion
   - et un traitement d'une série de signaux de rythme cardiaque consistant en un filtrage de passe-bande des fréquences comprises entre 0,04 et 0,26 Hz et de détection de pics et de mesure temporelle inter-pic RR, sur une seconde fenêtre temporelle glissante dont le résultat fourni une valeur $S_{valence}$ représentative de la positivité et la négativité de l'émotion.

2. Système selon la revendication 1 **caractérisé en ce que** lesdites électrodes (10) sont constitués par des plots formés par une feuille d'acier estampée.

**3.** Système selon la revendication 1 **caractérisé en ce qu'**il comporte en outre un capteur de température (33).

**4.** Système selon la revendication 1 **caractérisé en ce que** ledit capteur de rythme cardiaque délivre des signaux physiologiques par photopléthysmographie PPG.

**Patentansprüche**

**1.** System (30), umfassend einen Rechner, einen Herzfrequenzsensor (34) und einen elektrodermalen Sensor, umfassend mindestens zwei Elektroden (10), die dazu konfiguriert sind, jeweils Kontakt zu einem Hautbereich zu haben, und eine elektronische Schaltung, umfassend einen Analog-Digital-Wandler und einen Prozessor, der dazu konfiguriert ist, computerausführbare Anweisungen, die P-Elektroden (10) zum Messen des zwischen Elektrodenpaaren fließenden Stroms zu polarisieren, auszuführen, auf die gemessenen elektrischen Signale eine digitale Vorverarbeitung anzuwenden und die Leitfähigkeit der Hautschicht zu bestimmen, wobei der Sensor ferner einen Beschleunigungsmesser umfasst, der elektrische Signale an den Prozessor liefert, wobei die Vorverarbeitung den Pegel der vom Beschleunigungsmesser gelieferten Signale berücksichtigt,
**dadurch gekennzeichnet, dass** der Rechner ein Programm ausführt, das Folgendes steuert:

- das Verarbeiten der vom Prozessor bereitgestellten digitalen Leitfähigkeitsdaten innerhalb eines ersten gleitenden Zeitfensters, wobei das Ergebnis einen für die Intensität einer Emotion repräsentativen Wert $S_{Erregung}$ liefert,
- und das Verarbeiten einer Reihe von aus einer Bandpassfilterung von Frequenzen zwischen 0,04 und 0,26 Hz, einer Zacken-Detektion und einer Zeitmessung zwischen Zacken RR bestehenden Herzfrequenzsignalen innerhalb eines zweiten gleitenden Zeitfensters, wobei das Verarbeitungsergebnis einen die Positivität und die Negativität von Emotionen repräsentativen Wert $S_{Wertigkeit}$ liefert.

**2.** System nach Anspruch 1 **dadurch gekennzeichnet, dass** die Elektroden (10) aus Pads bestehen, die aus einem gestanzten Stahlblech gebildet sind.

**3.** System nach Anspruch 1 **dadurch gekennzeichnet, dass** es ferner einen Temperatursensor (33) umfasst.

**4.** System nach Anspruch 1 **dadurch gekennzeichnet, dass** der Herzfrequenzsensor (34) physiologische Signale durch Photoplethysmographie PPG liefert.

**Claims**

**1.** A system (30) comprising a computer, a heart rate sensor (34) and an electrodermal sensor comprising at least two electrodes (10), each of which is configured to be in contact with a dermal region, and an electronic circuit having an analog-to-digital converter and a processor that is configured to execute computer-executable instructions in order to bias said electrodes (10), to measure the current flowing between pairs of electrodes, to apply digital preprocessing to the measured electrical signals and to determine the conductance of the dermal layer, said sensor further comprising an accelerometer, delivering electrical signals to said processor, said preprocessing taking into account the level of said signals that are delivered by said accelerometer,
**characterized in that** said computer executes a program controlling:

- the processing of the digital conductance data provided by said processor over a first sliding time window, the result of which provides a value $S_{arousal}$ representative of the intensity of an emotion
- and processing of a series of heart rate signals consisting of bandpass filtering of the frequencies between 0.04 and 0.26 Hz and detecting peaks and measuring the time between the peaks RR, over a second sliding time window, the result of which provides a value $S_{valence}$ representative of the positivity and negativity of the emotion.

**2.** The system according to claim 1, **characterized in that** said electrodes (10) are formed by studs formed by a stamped steel sheet.

**3.** The system according to claim 1, **characterized in that** it further comprises a temperature sensor (33).

4. The system according to claim 1, **characterized in that** said heart rate sensor (34) delivers physiological signals by photoplethysmography (PPG).

[Fig. 1]

[Fig. 2]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3063425
- WO 2017202626 A
- US 2013183646 A
- KR 20160085577

**Littérature non-brevet citée dans la description**

- **C.G. JUNG.** *Studies in Word Analysis*
- Pervasive and unobtrusive émotion sensing for human mental health. **RUI GUO et al.** PERVASIVE COMPUTING TECHNOLOGIES FOR HEALTHCARE (PERVASIVEHEALTH), 2013 7TH INTERNATIONAL CONFERENCE ON. IEEE, 05 Mai 2013, 436-439
- What's Your Current Stress Level? Détection of Stress Patterns from GSR Sensor Data. **JORN BAKKER et al.** DATA MINING WORKSHOPS (ICDMW), 2011 IEEE 11 TH INTERNATIONAL CONFERENCE ON. IEEE, 11 Décembre 2011, 573-580